# EUROPEAN PATENT SPECIFICATION

(11) **EP 0 384 144 B1**
(45) Date of publication and mention of the grant of the patent: **03.04.1996**
(21) Application number: 90101405.0
(22) Date of filing: 24.01.1990
(51) Int. Cl.: C12P 21/08, A61K 39/395

(54) **Pharmaceutical composition for diseases caused by vasoconstriction**
Pharmazeutische Zusammensetzung gegen durch Vasokonstriktion hervorgerufene Krankheiten
Composition pharmaceutique contre les maladies provoquées par la vasoconstriction

(30) Priority: 26.01.1989 JP 17197/89; 05.12.1989 JP 315979/89
(43) Date of publication of application: 29.08.1990
(73) Proprietor: KOWA COMPANY, LTD., Naka-ku Nagoya-shi Aichi-ken (JP)
(72) Inventor: Okabe, Tetsuro, Bunkyo-ku, Tokyo (JP); Ohkuchi, Masao, Tokorozawa-shi, Saitama (JP); Ohhira, Akiyoshi, Sayama-shi, Saitama (JP); Kondo, Shoichi, Higashiyamato-shi, Tokyo (JP); Koshi, Tomoyuki, Shiki-shi, Saitama (JP); Edano, Toshiyuki, Higashimurayama-shi, Tokyo (JP); Hirata, Mitsuteru, Iruma-gun, Saitama (JP)
(74) Representative: Wächtershäuser, Günter, Prof. Dr.

(56) References cited:
- EP-A- 331 100
- EP-A- 0 423 333
- PROCEEDINGS OF THE NATL. ACADEMY OF SCIENCES OF THE UNITED STATES OF AMERICA, vol. 86, no. 8, April 1989, Baltimore, MD, US; A. INOUE et al.: "The human entothelin family: Three stucturally and pharmacologically distinct isopeptides predicted by three separate genes", pp. 2863-2867
- NATURE, vol. 332, no. 6163, March 31, 1988, London; M. YANAGISAWA et al.: "A novel potent vasoconstrictor peptide prduced by vascular endothelial cells", pp. 411-415

## Description

### BACKGROUND OF THE INVENTION

### Field of the Invention:

This invention relates to a novel pharmaceutical composition, and, more particularly, to a pharmaceutical composition comprising an anti-endothelin antibody as an effective component and useful for curing diseases caused by vasoconstriction.

### Description of the Background:

Hypertension, coronary artery contracture, basiler artery contracture, asthma, and the like are diseases caused by vasoconstriction. Noradrenalin, adrenaline, serotonin, thrombin, thromboxane As, prostaglandin F_{2α}, neuropeptide, oxyhemoglobin, and the like are known as substances which cause such diseases.

In recent years, two types of vasoconstriction, i.e., vascular endothelium-dependent vasoconstriction and vascular endothelium-independent vasoconstriction, have been studied separately. Existence of vasoconstrictors other than the above-mentioned substances has been suggested for vascular endothelium-dependent vasoconstriction caused by anoxia [Vanhoutte et al., *Circ. Res. 51,* 439-447 (1982)]. Following this, the existence of a peptide-like vasoconstrictive compound was confirmed in a supernatant of bovine aorta endothelial culture cells [Highsmith et al., *Am. J. Physiol., 284,* H432-437 (1985)]. This compound was revealed to be a peptide consisting of 21 amino acids. Further, the structure of this compound was determined and its cDNA was cloned. The compound was named endothelin-1 [Yanagisawa et al., *Nature, 332,* 411-415 (1988)]. There are other compounds having similar vasoconstrictive activities as endothelin-1. Such compounds include endothelin-2, endothelin-3 [Akihiro Inoue et al., *Proc. Natl. Acad. Sci.,* USA, *86,* 2863 (1989)], VIC [vasoactive intestinal contractor; Nario Ishihara et al., *FEBS Letters, 247,* 337 (1989)], and sarafotoxins S6ₐ₁, S6ₐ₂, S6_{b}, and S6 [Chikahisa Takahashi et al., *Toxicon, 26,* 543 (1988)]. They are called endothelin family compounds.

These compounds exhibit smooth muscle constrictive activities stronger than any other known vasoconstrictors, i.e., about 100 times as strong as angiotensin II. The compounds were discovered to be produced by endothelial cells alone and that their production was accelerated on endothelial cells at an mRNA level by the action of thrombin or adrenaline. Their mRNA level was also found to be higher in culture cells than in vascular endothelial cells [Yanagisawa et al., *Experimental Medicine, 6,* 297-302 (1988)]. Administration of an endothelin to an animal, on the other hand, was found to exhibit vasopressor in the animal [Watanabe et al., 73th Kinki-Branch Meeting, Japan Association of Pharmacology, (1988)], and to lower the blood circulation and induce ischemia [Ashino et al., 31st Meeting of Kidney Society of Japan (1988)]. A literature reports that a large dose administration induces cardiac failure which may result in the death of the subject [Yanagisawa et al., *Nature,* 335, 303 (1988)].

Based on the above facts, it can be safely judged that the production of an endothelin in a living body causes such diseases as hypertension, coronary artery contracture, basilar artery contracture, asthma, and the like.

Polyclonal antibodies and monoclonal antibodies having a specific affininty for endothelin are described in EP-A-033100 and in EP-A-0 423 333 which is to be considered pursuant to Art.54/3) EPC. The lattler document discloses that monodonal anti-endothelin antibodies can neutralize the action of endothelin in vivo and mentions its possible use as a phamaceutical.

There has been no medicine, however, which possesses a specific activity toward endothelins and can thus be used for curing the diseases caused by endothelins. Development of such a medicine has been strongly desired.

The present inventors have undertaken extensive studies for the development of such a medicine and found that antibodies of endothelins were effective for curing diseases caused by vasoconstriction. This finding has led to the completion of the present invention.

### SUMMARY OF THE INVENTION

Accordingly, an object of the present invention is to provide a pharmaceutical composition comprising an anti-endothelin antibody as an effective component and useful for curing diseases caused by vasoconstriction.

Other objects, features and advantages of the invention will hereinafter become more readily apparent from the following description.

### BRIEF DESCRIPTION OF THE DRAWINGS

Figure 1 shows the changes in blood pressure, heart rate, cardiac output, and total peripheral blood vessel resistance over time in rats which were administered endothelin-1, with or without the followed administration of anti-endothelin-1-polyclonal rabbit IgG.

Figure 2 shows the rates of endothelin-1 absorption by the endothelin-1-antibody prepared in Example 1.

Figure 3 shows the rates of endothelin-1 absorption by the anti-endothelin-1-monoclonal antibody prepared in Example 2.

### DETAILED DESCRIPTION OF THE INVENTION AND PREFERRED EMBODIMENTS

Anti-endothelin antibodies used as an effective component of the pharmaceutical composition of the present invention may be either monoclonal or polyclonal antibodies. Monoclonal antibodies include mouse monoclonal antibodies, human monoclonal antibodies, and monoclonal antibodies prepared by genetic engineering techniques.

Polyclonal antibodies used in the present invention can be prepared by a conventional immunological process. A preferable example of such a process comprises combining an endothelin antigen with another protein, administering the combined protein to an animal, collecting serum from the immuned animal, and isolating an antibody having a specificity against endothelins.

Endothelins which are the antigens used in the process can be prepared, for example, by culturing porcine endothelial cells under serum-free conditions followed by separation and purification of the target antigen from the supernatant [Yanagisawa et al., *Nature, 332,* 411-415 (1988)]. A peptide synthesis method can also be used for the preparation of such antigens [Yanagisawa et al., *Proc. Natl. Acad. Sci.,* USA, *85,* 6964 (1988)].

Thyroglobulin, bovine serum albumin, myoglobin, or the like can be used as a protein to combine with an endothelin.

An endothelin-protein conjugate can be prepared by condensing both by the formation of a peptide bond, or by combining an amino group of the endothelin and that of the protein through an alkylenedicarbonyl group, alkanediylidene group, phenylenedithiocarbamoyl group, or the like.

A conventional peptide synthesis method can be used for the condensation of an endothelin and a protein by the formation of a peptide bond, e.g. by reacting the endothelin and protein in a solvent, preferably in a buffer solution, in the presence of a condensing agent. As a condensing agent, 1-(3-dimethylaminopropyl)-3-ethylcarbodiimide, dicyclohexylcarbodiimide, or the like can be used.

A preferable group which bridges an amino group of an endothelin and that of a protein is an alkylenedicarbonyl group or an alkanediylidene group having 2-8 carbon atoms, phenylenedithiocarbamoyl, or the like. The carbon chain of the alkylenedicarbonyl or alkanediylidene may be either linear or branched, and may contain unsaturated bonds therein. Agents used for the bridging reaction are, for example, bis(sulfosuccinimidyl)suberate, disuccinimidylsuberate, or the like for the alkylenedicarbonyl group, glutalaldehyde or the like for the alkanediylidene group, and p-phenylenediisothiocyanate or the like for the phenylenedithiocarbamoyl group.

Purification of the combined endothelin-protein conjugate thus prepared can be carried out according to a conventional method. One of the preferable methods is dialyzing the product against a phosphate buffer containing a physiological saline. It is possible to obtain the target immune source in a powdery form from the dialyzed solution. More preferably, however, the dialyzed solution is used in the following step as is.

There are no specific restrictions as to the method by which the above immune source is administered to an animal to immunize it. Any conventional method can be used. Animals which can be used for the immunization are, for example, mouse, rat, guinea pig, rabbit, goat, and the like. The immune source is emulsified in a complete Freund's adjuvant, incomplete Freund's adjuvant, alum adjuvant, or the like, and is subcutaneously, intradermally, or intraperitoneally administered. It is desirable to administer the immune source at an interval of 1-5 weeks for a period of 1-20 months to produce the antibody. Exsanguination according to a conventional method is performed when the antibody titer goes up, followed by separation of serum, thus obtaining anti-serum containing the antibody.

The anti-serum is then purified by a known method. Salting-out using a neutral salt such as ammonium sulfate, sodium sulfate, or the like; alcohol precipitation, a precipitation method using polyethylene glycol, a heavy metal, or the like; electrophoresis, ion-exchange chromatography, gel filtration, affinity column chromatography, or the like is used independently or in combination for the purification. Since anti-endothelin antibodies of the present invention are those belonging to the IgG class, the use of a purification method capable of specifically separating such antibodies, e.g. gel filtration, affinity column chromatography using protein A, or the like, is preferable. In order to specifically eliminate an antibody against the protein which is combined with the endothelin, the use of affinity chromatography using a carrier to which the protein is combined is preferable.

Monoclonal antibodies of the present invention can also be prepared by a conventional method. A typical process comprises immunizing a mouse in the same way as in the preparation of polyclonal antibody using the same or similar immune source, collecting the antibody-producing cells, hybridizing them with mouse myeloma, selectively growing the hybridoma, detecting the antibody-producing hybridoma, cloning and culturing the hybridoma, and collecting the monoclonal antibody.

Suitable human monoclonal antibodies which can be used are those prepared by a process using a human-human hybridoma, a process using a human-mouse hetero-hybridoma, a process using a human-mouse-human triple-hybridoma, or a process using established cells which are transformed using Epstein-Barr virus.

The pharmaceutical composition of the present invention is very effective for curing diseases caused by vasoconstriction such as hypertension, coronary artery contracture, basilar artery contracture, and the like. Endothelin antibodies which are the active components of the pharmaceutical composition of the present invention exhibited an LD₅₀ 200 mg/kg or more value in acute toxicity tests, in which they are intravenously administered to rats, thus demonstrating that they can safely be administered to human.

The dose to be administered varies depending on the subject and his symptom. In general, it is desirable to administer the composition in an amount of 0.01-3,000 mg per day at one time or divided in 2-4 times. A preferable manner of administration is injection, although to direct administration to the rectum is acceptable. Injection preparations are prepared by dissolving the antibody in a buffer and optionally adding a stabilizer, a solubilizing agent, and the like. The preparations are then filled in vials and intravenously, subcutaneously, or intramuscularly injected. The antibody filled in vials and lyophillized may be used after dissolution into water for injection or physiological saline.

Other features of the invention will become apparent in the course of the following description of the experimental examples showing the curing effect by the use of the pharmaceutical composition and the examples for the preparation of the antibody of the present invention, which are given for illustration of the invention and are not intended to be limiting thereof.

### EXAMPLES

### Experimental Example 1

### Inhibition of Endothelin-Induced Contraction of Rat Isolated Aorta

Wistar male rats weighing 200-300 g were decapitated and the thoratic aorta was isolated. The aorta was cut into rings of about 3 mm width. An organic bath filled with 3 ml of a Krebs-Henseleit solution was aerated with a mixed gas (95% 0₂, 5% CO₂) and maintained at 37°C. The aorta segments were mounted between hooks, resting tension was adjusted to 2 g, and equilibration for 1 hour was followed by the maximum response to potassium chloride (80 mM), until a steady response was obtained. Anti-endothelin-1-mono- or polyclonal rabbit IgG was dissolved in the Krebs-Henseleit solution, and added to the organic bath. After 10 minutes, endothelin-1 or -2 dissolved in a 10 mM phosphate buffer containing 0.1% bovine serum albumin was added to the final concentration of 10 ng/ml, thus causing the aorta to constrict. Each constrictive reaction was isometrically measured and recorded. The constriction caused by endothelin-1 or endothelin-2 was measured 30 minutes after the administration and their rates of constriction were determined as a ratio of the maximum constriction in 80 mM potassium chloride. The results obtained for 6 trials for each treatment are averaged in Table 1.

From the results, it can be concluded that anti-endothelin-1-monoclonal or -polyclonal rabbit IgG effectively and dose-dependently inhibits endothelin-1 and endothelin-2-induced contraction in rat isolated thoracic aortae.

**TABLE 1**

| Treatment | Constriction Rate (%) | |
|---|---|---|
| | Endothelin-1 | Endothelin-2 |
| Untreated | 61 | 66 |
| Anti-endothelin-1-polyclonal rabbit IgG 100 µg/ml | 42 | 20 |
| Anti-endothelin-1-polyclonal rabbit IgG 300 µg/ml | 23 | 10 |
| Anti-endothelin-1-monoclonal rabbit IgG 0.3 µg/ml | 34.5 | - |
| Anti-endothelin-1-monoclonal rabbit IgG 1 µg/ml | 2.7 | - |

### Experimental Example 2

### Inhibition of Endothelin-Induced Contraction of Canine Isolated Arteries

Mongrel, male, adult dogs weighing about 9-13 kg were anesthetized with sodium pentobarbital (300 mg/kg, iv.). Their basilar, coronary, and kidney arteries were isolated and cut into rings of about 3 mm width. An organic bath filled with 3 ml of a Krebs-Henseleit solution was aerated with a mixed gas (95% O₂, 5% CO₂) and maintained at 37°C. The rings were mounted between hooks, resting tension was adjusted to 2 g, and equibration for 1 hour was followed by the maximum response to potassium chloride (80 mM), until a steady response was obtained. Anti-endothelin-1-polyclonal rabbit IgG was dissolved in the Krebs-Henseleit solution and added to the organic bath to the final concentration of 100 or 300 µg/ml. After 10 minutes, endothelin-1 or -2 dissolved in a 10 mM phosphate buffer containing 0.1% bovine serum albumin was added to the final concentration of 10 or 30 ng/ml (for both endothelin-1 and endothelin-2), thus causing the arteries to constrict. Each constriction reaction was isometrically measured and recorded. The constriction caused by endothelin-1 or endothelin-2 was measured 30 minutes after the administration and their rates of constriction were determined as the ratio of the maximum constriction in 80 mM potassium chloride. The results obtained for 4 trials for each treatment are averaged in Table 2.

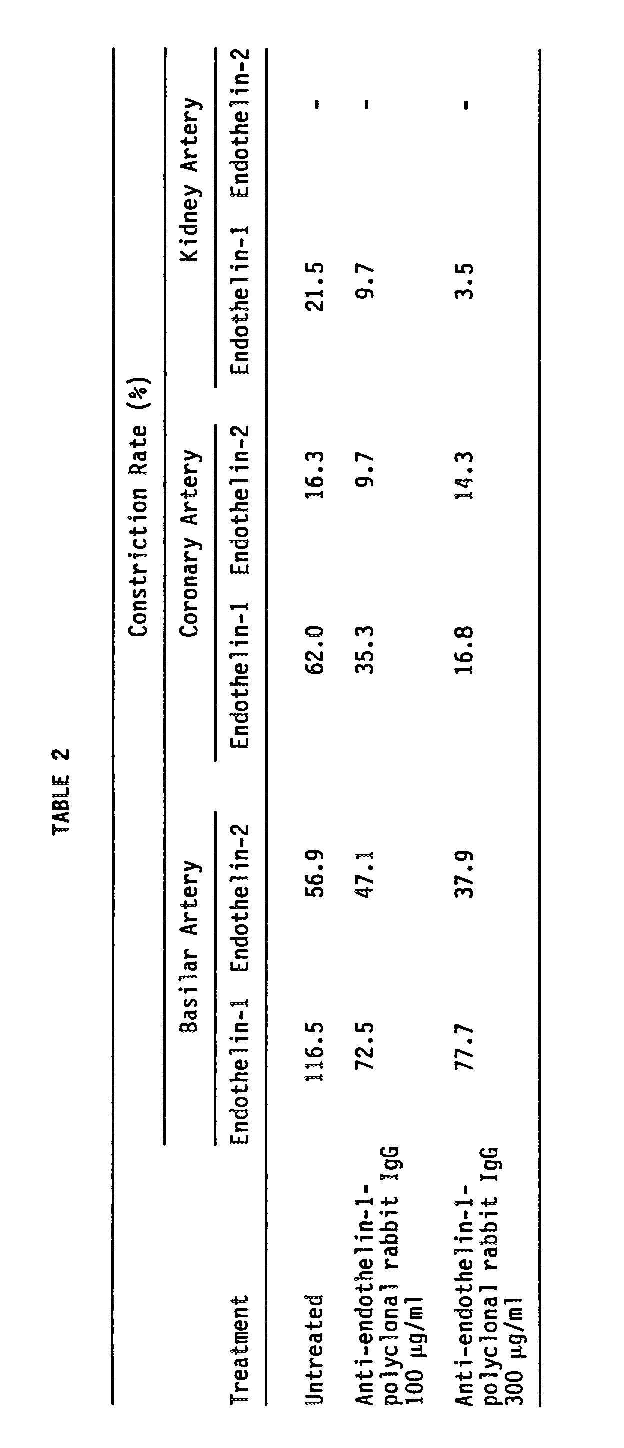

The results shown in Table 2 demonstrate that anti-endothelin-1-polyclonal rabbit IgG effectively inhibits constriction in canine isolated basilar, coronary, and kidney arteries induced by endothelin-1 and endothelin-2.

Anti-endothelin-1-monoclonal antibody prepared in Example 2 hereinafter also effectively inhibited contraction of canine isolated basilar, coronary, and kidney arteries induced by endothelin-1 and endothelin-2.

### Experimental Example 3

### Inhibition of Endothelin-1-Induced Cardiovascular Changes in Anesthetized Rats

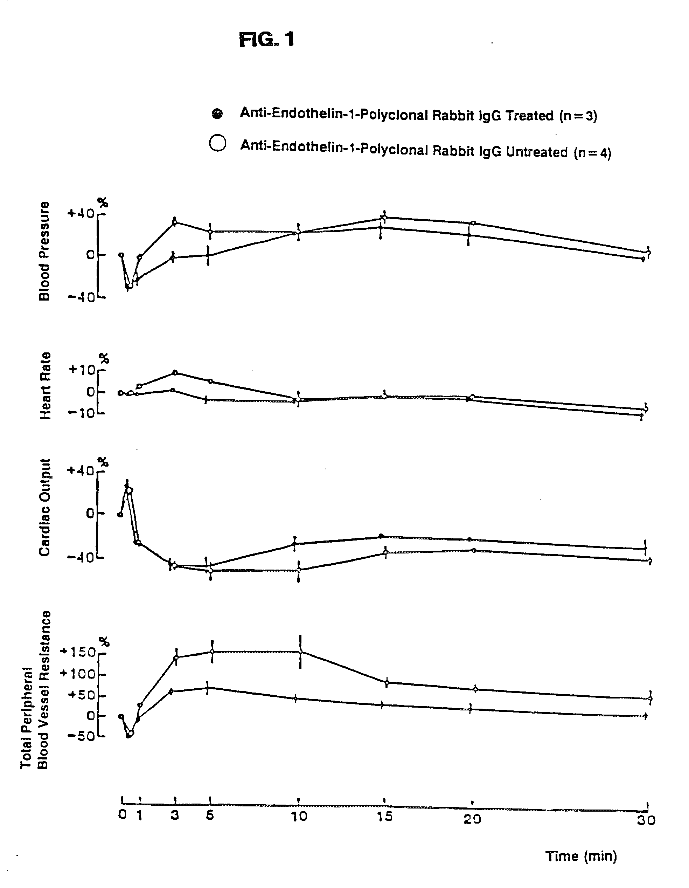

Blood pressure, heart rate, cardiac output, and total peripheral vascular resistance in Wistar male rats weighing 150-200 g anesthetized with urethane were measured to detect changes in these parameters by the administration of anti-endothelin-1-polyclonal rabbit IgG. Endothelin-1 dissolved in a 10 mM phosphate buffer containing 0.1% bovine serum albumin was intravenously injected at a dose of 0.3 nM/kg. Anti-endothelin-1-polyclonal rabbit IgG was dissolved in physiological saline and intravenously injected at a dose of 16.5 mg/kg 5 minutes before the injection of endothelin-1. Changes in the above parameters are shown in Figure 1.

Blood pressure in anti-endothelin-1-polyclonal rabbit IgG untreated groups decreased temporarily, followed by a prolonged increase. In anti-endothelin-1-polyclonal rabbit IgG treated groups, on the other hand, significant suppression of the blood pressure rise was observed, although the blood pressure rose continuously after a temporary decrease. Heart rate increased by the administration of endothelin-1 in anti-endothelin-1-polyclonal rabbit IgG untreated groups, but the increase was suppressed in the antibody treated groups. Cardiac output in both groups exhibited a decrease after a temporary rise, although the antibody administration demonstrated significant suppression of the rise. Total peripheral vascular resistance in antibody untreated groups was administered temporarily decreased and then continuously went up by administration of endothelin-1. The rise, however, was significantly suppressed in the antibody treated groups.

Anti-endothelin-1-polyclonal rabbit IgG exhibited the same effects against endothelin-2. Anti-endothelin-1-monoclonal antibody prepared in Example 2 hereinafter also exhibited the same effects as anti-endothelin-1-polyclonal rabbit IgG.

### Reference Example 1

Into 4.4 ml of 0.05 M phosphate buffer (pH 7.4) 1.1 mg (440 nM) of a human endothelin and 4.4 mg (6.67 nM) of thyroglobulin were dissolved. 110 mg of 1-(3-dimethylaminopropyl)-3-ethylcarbodiimide hydrochloride was added to the solution and the mixture was stirred overnight at room temperature. The reaction mixture was dialyzed against a 0.01 M phosphate buffer (pH 7.4) containing 0.15 M sodium chloride at 4°C for 2 days to obtain 8.5 ml of a phosphate buffer which contained endothelin-1-thyroglobulin conjugate (0.52 mg protein/ml).

The solution was frozen and stored. The required amount of the frozen material was taken out and thawed for use from time to time.

### Reference Example 2

Into 440 µl of a 0.05 M phosphate buffer (pH 7.4) 110 µg (44 nM) of endothelin-1 and 440 µg (6.67 nM) of bovine serum albumin were dissolved. 11 mg of 1-(3-dimethylaminopropyl)-3-ethylcarbodiimide hydrochloride was added to the solution and the mixture was stirred overnight at room temperature. The reaction mixture was dialyzed against a 0.01 M phosphate buffer (pH 7.4) containing 0.15 M sodium chloride at 4°C for 2 days to obtain 0.9 ml of a phosphate buffer which contained endothelin-1-bovine serum albumin conjugate (0.49 mg protein/ml).

### Reference Example 3

Into 500 µl of a 0.05 M phosphate buffer (pH 7.4) 100 µg (40 nM) of endothelin-1 and 500 µg (7.58 nM) of bovine serum albumin were dissolved. 80 µl of 1% aqueous solution of glutalaldehyde was added to the solution and the mixture was stirred at room temperature for 23 hours. The reaction mixture was dialyzed against a 0.1 M phosphate buffer (pH 7.42) containing 0.15 M sodium chloride at 4°C for 2 days to obtain 1 ml of a phosphate buffer which contained endothelin-1-bovine serum albumin conjugate cross-linked with a 1,5-pentanediylidene group (0.5 mg protein/ml).

### Reference Example 4

Into 500 µl of 0.05 M phosphate buffer (pH 7.4) 100 µg (40 nM) of endothelin-1 and 500 µg (7.58 nM) of bovine serum albumin were dissolved. A solution of 456 µg of dimethylformamide in 80 µl of bis(sulfosuccinimidyl)suberate was added to the solution and the mixture was stirred at room temperature for 23 hours. The reaction mixture was dialyzed against a 0.01 M phosphate buffer containing 0.15 M sodium chloride at 4°C for 5 days to obtain 1.4 ml of a phosphate buffer which contained endothelin-1-bovine serum albumin combination cross-linked with a 1,6-hexamethylenedicarbonyl group conjugate (0.36 mg protein/ml).

### Reference Example 5

Into 100 µl of 50% aqueous N-methylmorpholine (adjusted to pH 9.5 with trifluoroacetic acid) 100 µg (40 nM) of endothelin-1 was dissolved. To the solution thus prepared was added 2.2 mg (11.4 µM) of p-phenylenediisothiocyanate in 200 µl of dimethylformamide, and the mixture was stirred at room temperature for 6 hours. After the addition of ethanol and ether, the mixture was centrifuged. Ether was added to the residue and the suspension was centrifuged. The precipitate was washed three times with ether, dried, and dissolved into a mixed solution of 50 µl of N-methylmorpholine-trifluoroacetic acid and 50 µl of dimethylformamide. To this solution was added a solution of 500 µg (7.58 nM) of bovine serum albumin in 100 µl of N-methylmorpholine-trifluoroacetic acid. The mixture was stirred at room temperature overnight under a nitrogen atmosphere, and, after a further addition of 100 µl of dimethylformamide, was stirred for a further 6 hours at room temperature. The resultant reaction mixture was dialyzed against a 0.01 M phosphate buffer (pH 7.5) containing 0.15 M sodium chloride for 4 days to obtain 1.1 ml of a phosphate buffer which contained endothelin-1-bovine serum albumin conjugate cross-linked by p-phenylenedithiocarbamoyl group.

### Example 1

To the phosphate buffer (pH 7.2) containing an endothelin-1-thyroglobulin conjugate prepared in Reference Example 1 was added the same amount of complete Freund's adjuvant to produce a homogeneous emulsion. The emulsion was subcutaneously injected into the foot pads of 3 rabbits at a dose of 500 µg/rabbit as protein to immunize the animals. After the initial immunization, the antigen emulsified with incomplete Freund's adjuvant was injected, and similarly the rabbits were boostered at two week intervals. After the immunization, blood was collected from the ear vein at one-week intervals. At eleven weeks after the antibody titer had gone up exsanguination was performed through the carotid artery to obtain anti-serum. 30 ml of saturated aqueous ammonium sulfate was added to 30 ml the anti-serum, and the mixture was left at 4°C for 2 hours and zcentrifuged at 7,500 rpm for 10 minutes. The precipitate was dissolved into a 10 mM phosphate buffer-physiological saline (pH 7.4) (hereinafter referred to as PBS) and dialyzed against PBS for 2 days. After dialysis, PBS was added to the dialyzate to adjust the total volume to 45 ml. This solution was subjected to thyroglobulin-celluofine affinity column chromatography (30 ml), thus obtaining 60 ml of a fraction which passed through the column without being absorbed. This fraction was subjected to protein A-celluofine affinity column chromatography (30 ml). The column was eluted with 30 ml of a 0.1 M glycin-HCl buffer (pH 2.7) containing 0.2 M sodium chloride. After neutralizing with 1 M Tris buffer, the eluent was dialyzed against deionized water for 2 days and lyophillized to produce 210 mg of an antibody (IgG) possessing specificity to endothelin-1 as white powder.

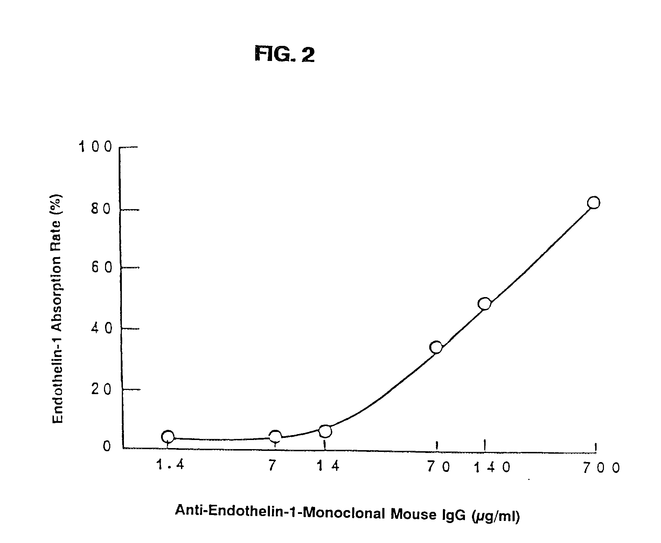

This IgG at a 7 mg/ml concentration had an antibody titer of 1:89,200 by the ELISA method. It absorbed 80% of 10 ng/ml endothelin-1 at a concentration of 700 µg/ml as shown in Figure 2.

### Example 2

To the phosphate buffer (pH 7.2) containing the endothelin-1-thyroglobulin conjugate prepared in Reference Example 1 was added the same amount of complete Freund's adjuvant to produce a homogeneous emulsion. The emulsion was subcutaneously injected into 3 BALB/c mice at a dose of 50 µg/mouse as protein to immunize the animals. Two weeks after the initial immunization, the antigen was emulsified with incomplete Freund's adjuvant and injected to the mice at a dose of 50 µg/mouse as protein to immunize them. Four weeks after the second immunization, a phosphate buffer (pH 7.2) containing the antigen was intraperitoneally injected into the mice at a dose of 50 µg/mouse as protein. Three days following this injection, antibody titers were measured by the ELISA method. Four days after the titer measurement, the spleen cells of the mouse which exhibited the highest titer (1:347,800) were isolated. The spleen cells and the mouse myeroma cells P3x63Ag 8U.1 were fused using ethylene glycol 1500. Subsequently, the titer for endothelin-1 of the hybridoma selected by HAT medium was measured and colonies having a higher antibody titer were cloned. A monoclonal antibody producing hybridoma BT-H51 having specificity to endothelin-1 was thus prepared. The hybridoma was a novel stock capable of producing the monoclonal antibody having specificity to endothelin-1 and was deposited with Fermentation Research Institute, Agency of Industrial Science and Technology (Deposition No. 2596, FERM BP-2596).

The hybridoma was cultured and intraperitoneally inoculated in a BALB/c mouse which had received an intraperitoneal injection of pristan 3 weeks beforehand. After 10-12 days, abdominal dropsy of the mouse was collected. To a 10 ml aliquot of the abdominal dropsy the equivalent amount of saturated ammonium sulfate solution was added, and the mixture was left at 4°C for 2 hours and centrifuged at 7,500 rpm for 10 minutes. The precipitate was dissolved into 15 ml of PBS and dialyzed against PBS for 2 days. After dialysis, PBS was added to the dialyzate to adjust the total volume to 15 ml. This solution was subjected to protein G-celluofine affinity column chromatography (10 ml). The column was eluted with 0.1 M glycin-HCl buffer (pH 2.7) containing 0.2 M sodium chloride. After neutralizing with 1 M Tris buffer, the eluent was lyophillized to produce 80 mg of an anti-endothelin-1-monoclonal antibody as white powder.

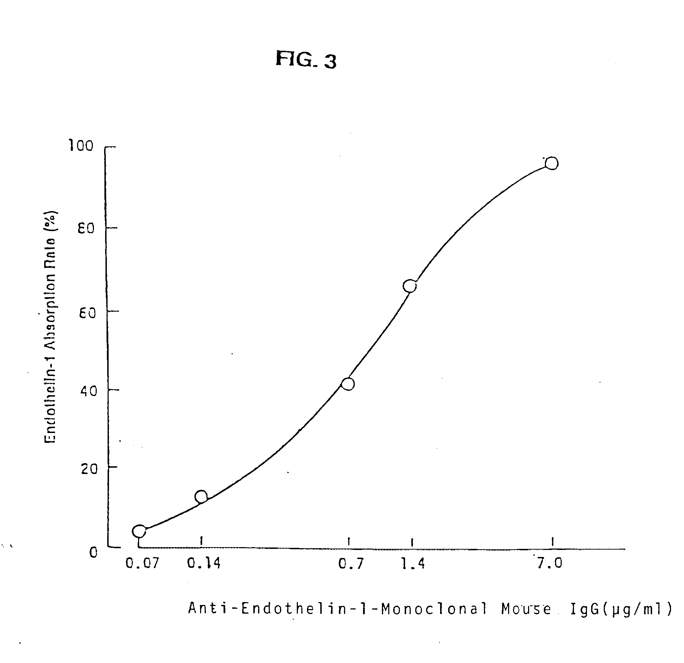

This IgG at a 7 mg/ml concentration had an antibody titer by the ELISA method of 1:102,400. It absorbed 80% of 10 ng/ml endothelin-1 at a concentration of 2.2 µg/ml as shown in Figure 3.

The anti-endothelin-1-monoclonal antibody thus prepared has the following characteristics, in which the molecular weight was measured by the SDS-polyacrylamide gel electrophoresis method. The isoelectric point was determined using Pharmacia-LKB electrophoresis equipment, and the immunoglobulin subclass was determined by the Ouchterlony immunodiffusion method.
(a) Molecular weight: 155,000 ± 5,000
(b) IgG Subclass: IgG₁
(c) Isoelectric point: PI 6.6-7.2
(d) Recognizes a loop portion (1-15) of endothelin-1 and the S-S bond configuration.

Obviously, numerous modifications and variations of the present invention are possible in light of the above teachings.

## Claims

1. A pharmaceutical composition for curing diseases caused by vasoconstriction, comprising an anti-endothelin antibody as an effective component.

## Patentansprüche

1. Arzneimittel zur Heilung von Erkrankungen, die durch Gefäßverengung hervorgerufen werden, umfassend einen Anti-Endothelin-Antikörper als eine wirksame Komponente.

## Revendications

1. Composition pharmaceutique pour traiter des maladies provoquées par la vasoconstriction, comprenant un anticorps anti-endothéline en tant que composant actif.
